# EUROPEAN PATENT APPLICATION

(11) **EP 3 964 265 A1**
(43) Date of publication of application: **09.03.2022**
(21) Application number: 20382792.8
(22) Date of filing: 07.09.2020
(51) Int. Cl.: A61P 37/04, C12N 5/077, C12N 15/86

(54) **MESENCHYMAL STEM CELLS CO-EXPRESSING CXCR4 AND IL-10 AND USES THEREOF**

(71) Applicant: Fundacion Instituto De Investigacion Sanitaria Fundacion Jimenez Diaz, 28040 Madrid (ES); Centro de Investigaciones Energéticas, Medioambientales y Tecnológicas O.A., M.P. (CIEMAT), 28040 Madrid (ES); Consorcio Centro de Investigación Biomédica en Red, M.P. (CIBER), 28029 Madrid (ES)
(72) Inventor: HERVÁS-SALCEDO, Rosario, 28040 Madrid (ES); GARCÍA-OLMO, Damián, 28040 Madrid (ES); GARCÍA-ARRANZ, Mariano, 28040 Madrid (ES); HERNANDO RODRÍGUEZ, Miriam, 28040 Madrid (ES); BUEREN RONCERO, Juan Antonio, 28040 Madrid (ES); YÁÑEZ GONZÁLEZ, Rosa María, 28040 Madrid (ES); FERNÁNDEZ, María, 28040 Madrid (ES)
(74) Representative: Hoffmann Eitle

(57) **Abstract**

The present invention refers to mesenchymal stem cells (MSCs) characterized in that they are transduced with an integrative expression vector in order to stably co-express the chemokine receptor type 4 CXCR4 and the interleukin IL-10. The present invention also refers to the use of said MSCs as a medicament, particularly in the treatment of inflammatory and/or autoimmune diseases.

SEQUENCE LISTING

<110> Fundación Instituto de Investigación Sanitaria Fundación Jiménez Díaz
(FIIS-FJD)
Centro de Investigaciones Energéticas, Medioambientales y
Tecnológicas, O.A., M.P. (CIEMAT)
Consorcio Centro de Investigación Biomédica en Red, M.P. (CIBER)

<120> MESENCHYMAL STEM CELLS CO-EXPRESSING CXCR4 AND IL-10 AND USES
THEREOF

<130> 905 345

<160> 3

<170> PatentIn version 3.5

<210> 1
<211> 1068
<212> DNA
<213> Artificial sequence

<220>
<223> Artificial sequence

<400> 1
atgtcaatcc ccctgccact gctgcagatc tacacaagcg ataactacac cgaagaaatg 60

ggctccggcg actacgactc tatgaaggag ccatgcttca gggaggaaaa cgccaatttc 120

aacaagatct ttctgcccac aatctacagc atcatttttc tgactggaat cgtgggaaat 180

ggcctggtca ttctggtcat gggctaccag aagaaactgc gatccatgac tgacaagtat 240

cggctgcacc tgtctgtcgc agatctgctg ttcgtgatca ccctgccatt ttgggccgtc 300

gacgccgtgg ctaattggta tttcggcaac tttctgtgca aagccgtcca cgtgatctac 360

acagtcaatc tgtatagctc cgtgctgatc ctggctttca ttagtctgga tcgctacctg 420

gcaattgtgc atgccactaa cagccagcgg cccagaaagc tgctggctga gaaagtggtc 480

tatgtcgggg tgtggatccc cgcactgctg ctgaccatcc ctgacttcat ttttgccaat 540

gtgagcgaag ctgacgatag gtacatttgt gaccgctttt atcctaacga tctgtgggtg 600

gtcgtgttcc agtttcagca catcatggtc gggctgattc tgccaggaat tgtgatcctg 660

tcatgctact gtatcattat cagcaagctg tcccactcta aaggccatca gaagcgaaaa 720

gccctgaaga ccacagtgat tctgatcctg gctttctttg catgctggct gccctactat 780

attgggatca gcattgattc cttcatcctg ctggagatta tcaagcaggg ctgtgagttt 840

gaaaataccg tgcacaaatg gatctccatt acagaagcac tggccttctt tcattgctgt 900

ctgaacccta tcctgtatgc tttcctgggc gcaaagttta aaacttccgc ccagcatgct 960

ctgaccagtg tgtcaagagg ctctagtctg aaaatcctgt ctaaggggaa aaggggcggg 1020

cactcaagcg tgtctacaga gagtgaatcc tctagtttcc attcaagc 1068

<210>  2
<211>  60
<212>  DNA
<213>  Artificial sequence

<220>
<223>  Artificial sequence

<400>  2
caatgtacta actacgcttt gttgaaactc gctggcgatg ttgaaagtaa ccccggtcct        60


<210>  3
<211>  537
<212>  DNA
<213>  Artificial sequence

<220>
<223>  Artificial sequence

<400>  3
atgcattcat ccgctctgct gtgctgtctg gtgctgctga ccggcgtgcg ggcaagtcca        60

ggccagggaa ctcagtccga aaactcctgc acccacttcc ccgggaacct gcctaatatg       120

ctgcgagacc tgcgggatgc ctttagcagg gtgaaaacat tctttcagat gaaggaccag       180

ctggataacc tgctgctgaa agagagcctg ctggaagact tcaagggcta cctggggtgt       240

caggctctgt ccgaaatgat ccagttttat ctggaggaag tgatgccaca ggcagagaac       300

caggaccccg acatcaaggc ccacgtcaac tctctgggag agaatctgaa gactctgagg       360

ctgcgcctgc ggagatgcca tagattcctg ccctgtgaga ataagtctaa agccgtggaa       420

caggtcaaaa acgcttttaa taagctgcag gagaaaggca tctacaaggc tatgagtgaa       480

ttcgacatct tcatcaacta catcgaggca tatatgacca tgaagattcg caattga          537

## Description

### FIELD OF THE INVENTION

The present invention refers to the medical field. Particularly, the present invention refers to mesenchymal stem cells (MSCs) characterized in that they are transduced with an integrative expression vector in order to stably co-express the chemokine receptor type 4 CXCR4 and the interleukin IL-10. The present invention also refers to the use of said MSCs as a medicament, particularly in the treatment of inflammatory and/or autoimmune diseases.

### PRIOR ART

MSCs are multipotent adult stromal cells with immunomodulatory effects on activated lymphoid cells, including T cells, B cells, natural killer cells, and dendritic cells. MSCs display the ability to home on inflamed sites, where they can modulate inflammatory reactions and contribute to the repair of injured tissues.

In animal models, MSCs have demonstrated their efficacy both in regenerative medicine and also in inflammatory and autoimmune disease models. In phase I/II clinical trials, MSCs have demonstrated a safety profile and showed preliminary evidence of clinical benefit in different diseases such as steroid-resistant graft versus host disease (GVHD), severe systemic lupus erythematosus, complex perianal fistulas, knee osteoarthritis or chronic complete paraplegia, among others. Despite the results obtained in animal models and early-phase clinical trials, only in three Phase III clinical trials the therapeutic efficacy of MSCs has shown statistical significance over standard therapies. These include the treatment of complex perianal fistulas (NCT00475410), steroid-refractory GVHD in children (NCT02336230) and chronic advanced ischemic heart failure (NCT01768702).

Among the parameters that may reduce the therapeutic efficacy of MSCs, it is worth mentioning that the ex vivo expansion of these cells has shown to reduce the modest expression of homing receptors observed in MSCs, and also to induce the senescence in these cells.

Consequently, the present invention is focused on improving the therapeutic efficacy of MSCs, particularly by improving the migration of MSCs towards inflamed sites and also by secreting immunosuppressive and anti-inflammatory cytokines, thus potentiating the therapeutic efficacy of standard unmodified MSCs.

### Description of the invention

### Brief description of the invention

As explained above, the present invention is focused on improving the therapeutic efficacy of MSCs, particularly by enhancing the migration of MSCs towards inflamed sites and by enhancing the release of immunosuppressive and anti-inflammatory cytokines as compared to standard unmodified MSCs.

In order to do so, the inventors of the present invention have used MSCs which have been transduced with an integrative expression vector co-expressing the chemokine receptor type 4 CXCR4 and the interleukin IL-10.

Particularly, a lentiviral vector encoding for CXCR4 and IL-10 was constructed in the context of the present invention. This expression vector was used for transducing MSCs thus co-expressing in a stable manner both CXCR4 and IL-10.

**Example 2.1** shows that MSCs transfected with a CXCR4-IL10 mRNA exert anti-inflammatory properties in a mouse model of local inflammation. Nevertheless, these cells do not show enhanced anti-graft versus host disease (GvHD) properties compared to WT MSCs **(Example 2.2).** In contrast to MSCs transfected with the CXCR4-IL10 mRNA, MSCs that had been transduced with a lentiviral vector carrying the CXCR4-IL10 sequence **(Example 2.3),** not only exerted enhanced *in vitro* immunomodulatory properties **(Examples 2.4** and **2.5)** and local *in vivo* anti-inflammatory effects compared to WT MSCs **(Example 2.3 -2.6),** but strikingly also developed a significant anti GvHD, as shown in **Example 2.7** of the present invention.

In fact, the *in vitro* experiments included in the present invention show that the stable co-expression of these molecules efficiently enhanced the migration of MSCs towards SDF-1 and improved the immunosuppressive properties of these cells. Moreover, the preferential homing of MSCs ectopically expressing CXCR4 and IL10 to inflamed pads was demonstrated in a mouse model in which a local pad inflammation was induced. Taken together, these results demonstrate that the stable co-expression of specific homing and anti-inflammatory molecules, such as CXCR4 and IL10, in human MSCs confers an enhanced anti-inflammatory potential in these cells compared to WT MSCs. The use of this new generation of MSCs transduced with an integrative expression vector co-expressing CXCR4 and IL10 will have a significant impact in clinical cell therapy for the treatment of inflammatory and/or autoimmune diseases.

Consequently, in summary, it is herein proposed the use of MSCs transduced with an integrative expression vector co-expressing both CXCR4 and IL-10 as a medicament, particularly in the treatment of inflammatory and/or autoimmune diseases.

So, the first embodiment of the present invention refers to an expression cassette (hereinafter the expression cassette of the invention) comprising a polynucleotide sequence which in turn comprises: a) a promoter, b) a sequence encoding the chemokine receptor type 4 CXCR4 and c) a sequence encoding interleukin IL-10. In a preferred embodiment, the expression cassette further comprises a regulatory element for increasing transgene expression. In a preferred embodiment, the regulatory element is the woodchuck hepatitis virus regulatory element (WPRE) RNA export signal sequence or a functional variant or fragment thereof. In a preferred embodiment, the expression cassette further comprises, between the sequence encoding the chemokine receptor type 4 CXCR4 and the sequence encoding interleukin IL-10, a sequence which encodes an autocatalytic peptide. In a preferred embodiment, the autocatalytic peptide is E2A. In a preferred embodiment the promoter is a human phosphoglycerate kinase (PGK) promoter sequence or a functional homolog or variant thereof. In a preferred embodiment, the expression cassette comprises in the order 5 ' to 3': a) a human phosphoglycerate kinase (PGK) promoter sequence or a functional homolog or variant thereof, b) a sequence encoding the chemokine receptor type 4 CXCR4, c) a sequence encoding the autocatalytic peptide E2A, d) a sequence encoding interleukin IL-10; and d) the woodchuck hepatitis virus post-transcriptional regulatory element (WPRE).

In a preferred embodiment, the expression cassette comprises codon optimized sequences of the human genes CXCR4 (SEQ ID NO: 1) and IL10 (SEQ ID NO: 3), using a sequence coding the autocatalytic peptide E2A (SEQ ID NO: 2) to ease the co-expression of both molecules.

The second embodiment of the present invention refers to a recombinant gene delivery vector (hereinafter the recombinant gene delivery vector of the invention) comprising the above defined expression cassette. In a preferred embodiment, the recombinant gene delivery vector is a lentiviral vector. In a preferred embodiment, the vector of the invention is an integrative vector which is permanently incorporated into the host chromosomes.

The third embodiment of the present invention refers to a cell (hereinafter the cell of the invention) comprising the expression cassette or the recombinant gene delivery vector of the invention. In a preferred embodiment, the cells are MSCs derived from bone marrow, placenta, umbilical cord, amniotic membrane, menstrual blood, peripheral blood, salivary gland, skin and foreskin, synovial fluid, amniotic fluid, endometrium, adipose tissue, cord blood and / or dental tissue.

The fourth embodiment of the present invention refers to a pharmaceutical composition comprising the recombinant gene delivery vector or the cell of the invention and, optionally, pharmaceutically acceptable excipients or carriers.

The fifth embodiment of the present invention refers to the gene delivery vector or the cells of the invention for use as a medicament. In a preferred embodiment, the present invention refers to the gene delivery vector or the cells of the invention for use in the treatment of inflammatory diseases and/or autoimmune diseases, for instance Graft-versus-host disease (GvHD), sepsis or rheumatoid arthritis. Alternatively, this embodiment refers to a method for treating inflammatory diseases and/or autoimmune diseases, for instance Graft-versus-host disease (GvHD), sepsis or rheumatoid arthritis, which comprises the administration to the patient of a therapeutically effective dose or amount of the gene delivery vector or the cells of the invention, or a pharmaceutical composition comprising thereof.

For the purpose of the present invention the following terms are defined:
- By "comprising" it is meant including, but not limited to, whatever follows the word "comprising". Thus, use of the term "comprising" indicates that the listed elements are required or mandatory, but that other elements are optional and may or may not be present.
- By "consisting of' it is meant "including, and limited to", whatever follows the phrase "consisting of'. Thus, the phrase "consisting of' indicates that the listed elements are required or mandatory, and that no other elements may be present.
- "Pharmaceutically acceptable excipient or carrier" refers to an excipient that may optionally be included in the pharmaceutical composition of the invention and that causes no significant adverse toxicological effects to the patient.
- By "therapeutically effective dose or amount" the present invention refers to the situation when the cells or the pharmaceutical composition are administered as described above and brings about a positive therapeutic response in a subject having an inflammatory or autoimmune disease. The exact amount required will vary from subject to subject, depending on the species, age, and general condition of the subject, the severity of the condition being treated, mode of administration, and the like. An appropriate "effective" amount in any individual case may be determined by one of ordinary skill in the art using routine experimentation, based upon the information provided herein.

### Brief description of the figures

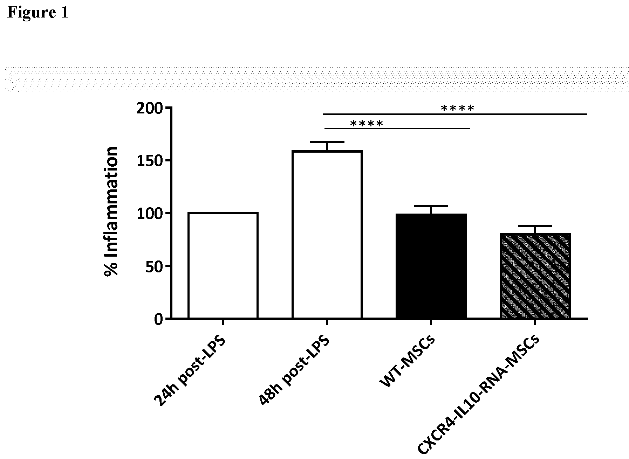

**Figure 1****. Evidence of *in vivo* efficacy of MSCs transfected with the bicistronic CXCR4-IL10 mRNA in a mouse model of local inflammation.** Enhanced anti-inflammatory effect of MSCs transfected with the CXCR4-IL10 mRNA is observed as compared to WT-MSCs.

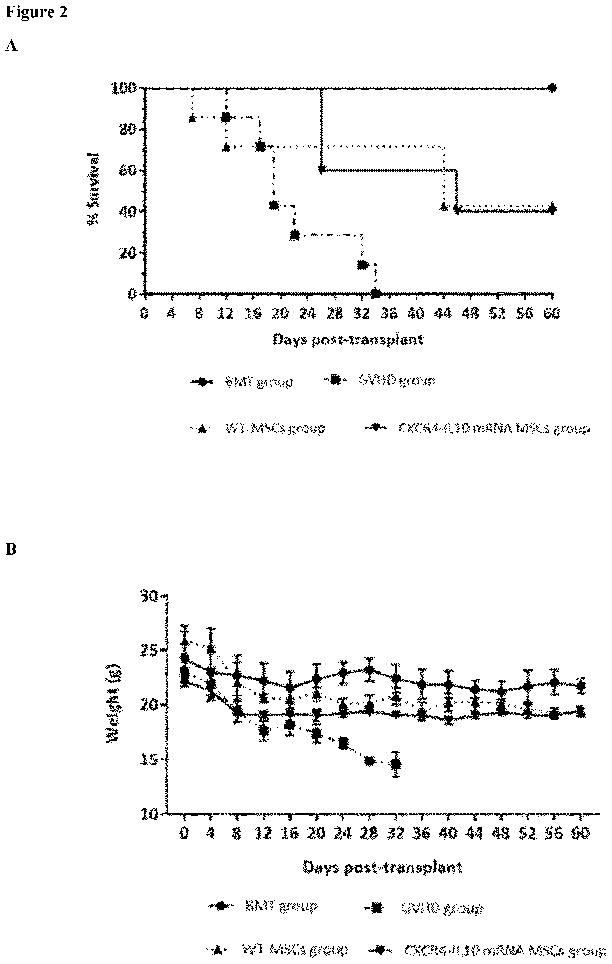

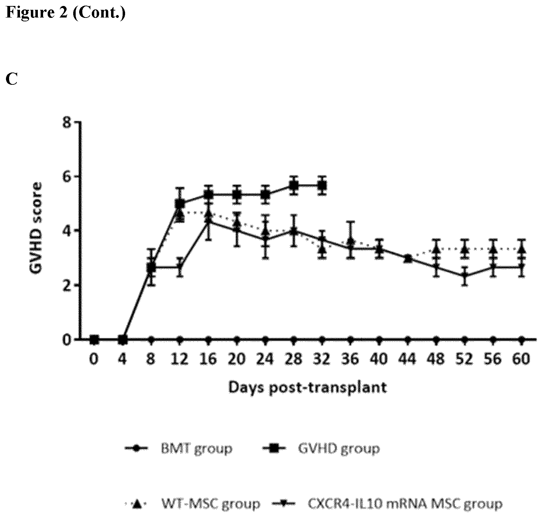

**Figure 2****. Absence of *in vivo* efficacy of MSCs transfected with the bicistronic CXCR4-IL10 mRNA in a mouse model of GVHD.** A) Survival curve B) Weight and C) Clinical score.

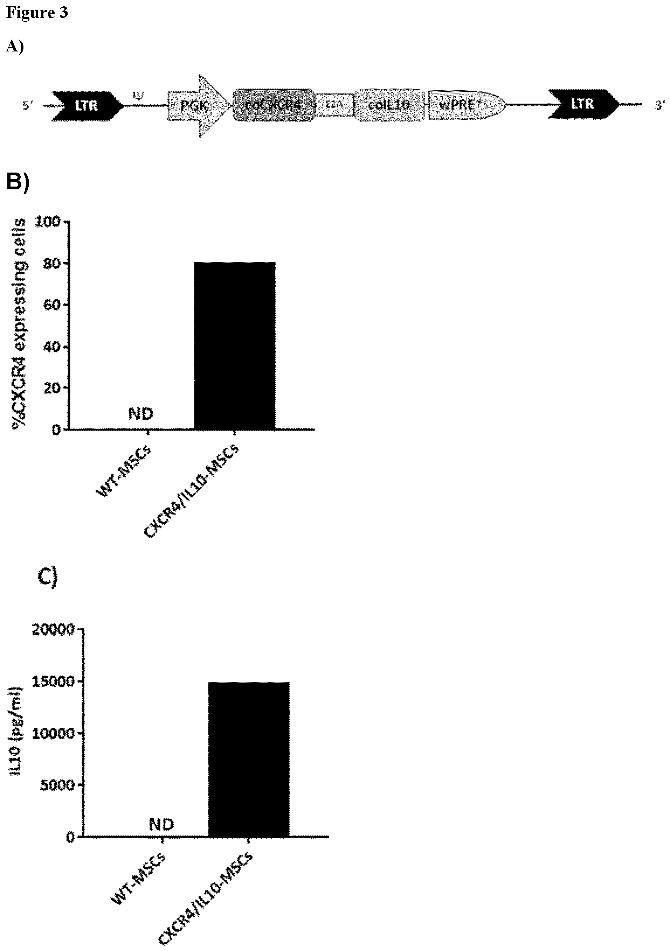

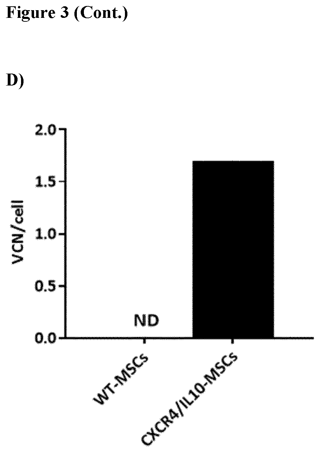

**Figure 3****. (A)** Design of the bicistronic lentiviral vector used to co-express CXCR4 and IL-10. **(B)** Levels of CXCR4, **(C)** IL-10 secretion and **(D)** vector copy number per cell (VCN/Cell) in CXCR4/IL10-MSCs compared to WT-MSCs. N.D. Not detectable

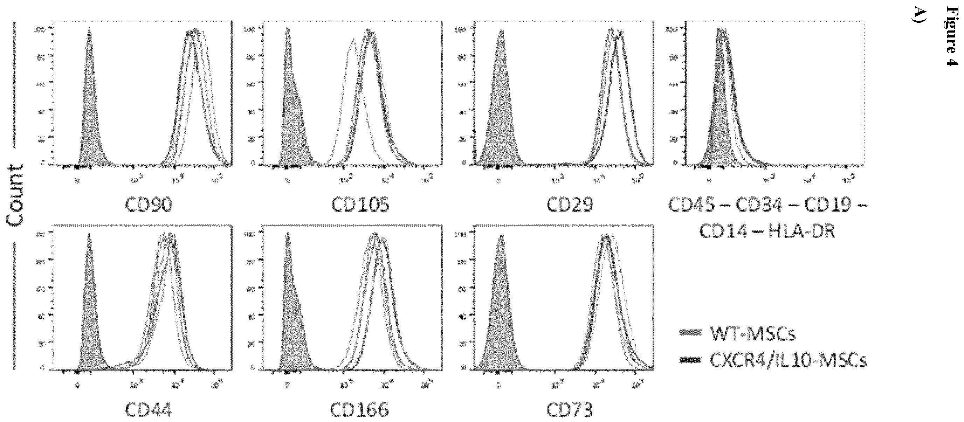

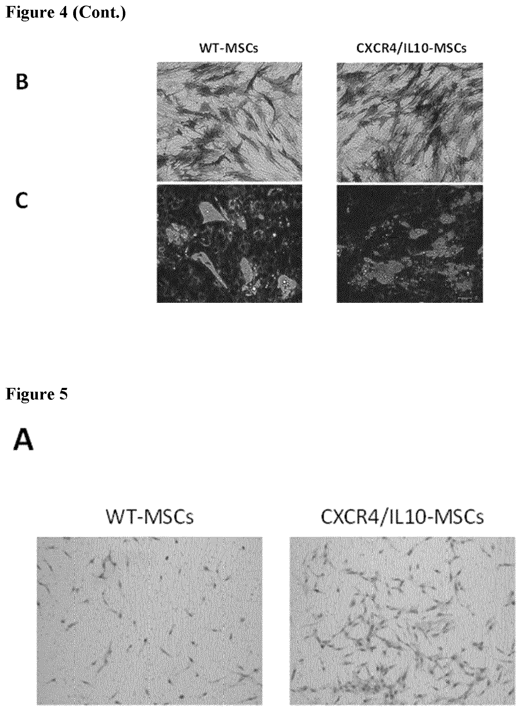

**Figure 4****. *In vitro* characterization of MSCs transduced with the PGK-CXCR4-IL10 lentiviral vector. A)** Immunophenotype of CXCR4/IL10-MSCs compared to WT-MSCs. **B)** Differentiation capacity of CXCR4/IL10-MSCs to bone tissue compared to WT-MSCs. **C)** Differentiation capacity of CXCR4/IL10-MSCs to adipose tissue compared to WT-MSCs.

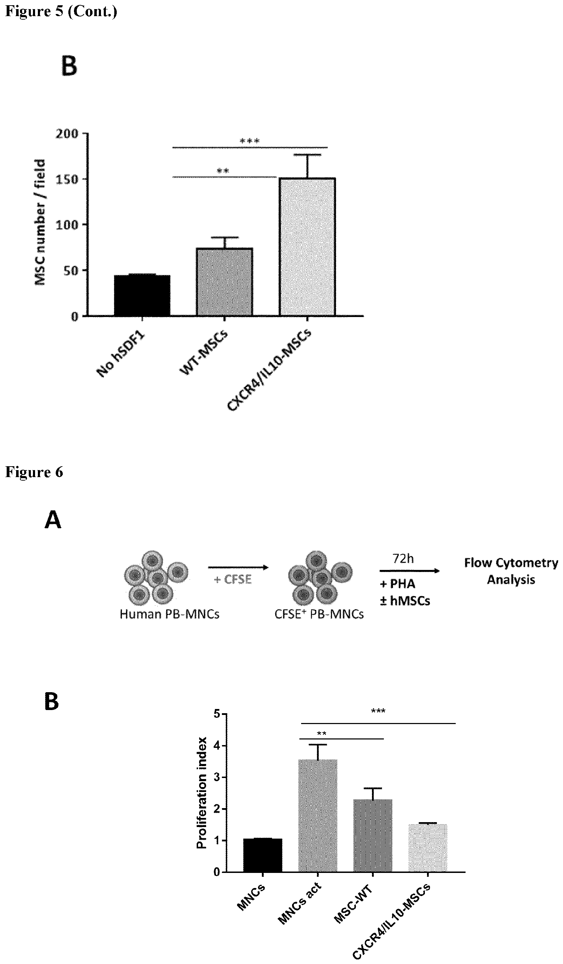

**Figure 5****. Enhanced migration capacity of MSCs transduced with PGK-CXCR4-IL10 lentiviral vector compared to WT-MSCs. A)** Representative picture of the migration ability of WT-MSCs and CXCR4/IL10-MSCs in response to SDF-1. **B)** Quantification of the migration ability of WT-MSCs and CXCR4/IL10-MSCs in response to SDF-1.
**Figure 6****. Enhanced *in vitro* immunosuppression capacity of MSCs transduced with the PG-CXCR4-IL10 lentiviral vector. A)** Scheme of the experimental system used to evaluate the *in vitro* immunosuppressive activity of MSCs. **B)** CXCR4/IL10-MSCs showed improved immunosuppression capacity than WT-MSCs.

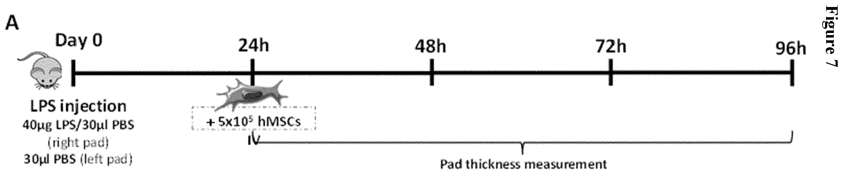

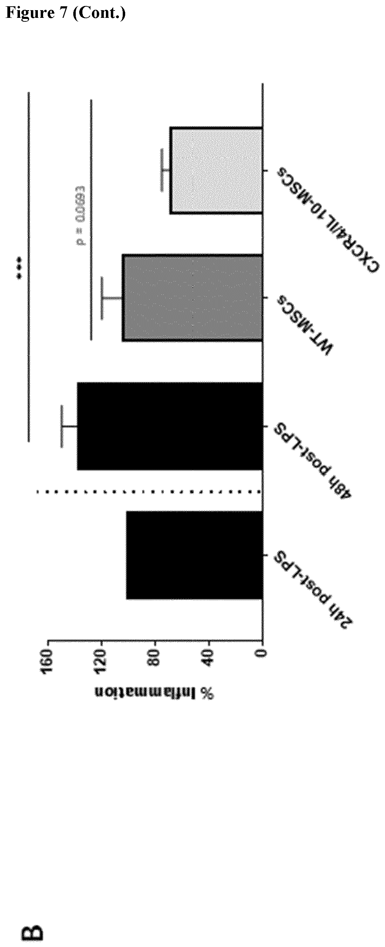

**Figure 7****. Enhanced *in vivo* efficacy of MSCs transduced with the PGK-CXCR4-IL10 lentiviral vector in a mouse model of local inflammation.** A) Scheme of the experimental system used to evaluate the *in vivo* anti-inflammatory effect of WT-MSCs and CXCR4/IL10-MSCs. **B)** Enhanced anti-inflammatory effect of MSCs transduced with the PGK-CXCR4-IL10 lentiviral vector compared to WT-MSCs.

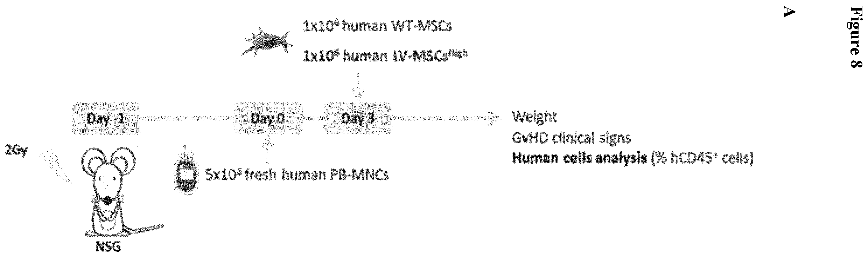

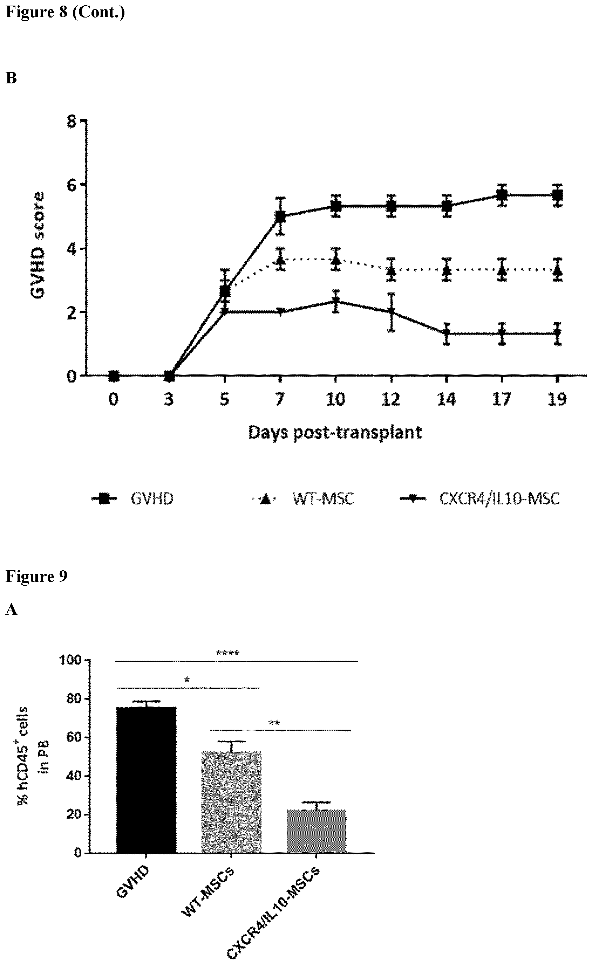

**Figure 8****. Enhanced anti-GvHD of MSCs transduced with the PGK-CXCR4-IL10 LV compared to WT-MSCs: Analysis of the GvHD clinical signs. A)** Scheme of the experimental system used to evaluate the *in vivo* anti-GVHD of WT-MSCs and CXCR4/IL10-MSCs. **B)** GVHD score comparing different experimental groups.

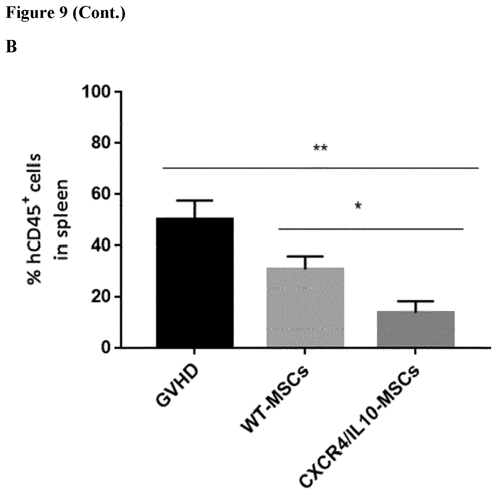

**Figure 9****. Enhanced anti-GvHD of MSCs transduced with the PGK-CXCR4-IL10 LV compared to WT-MSCs: A)** Flow cytometry analysis of human CD45⁺ cells in peripheral blood of recipient mice showing a reduced expansion of xenogenic donor leukocyte in the GVHD humanized mouse model. **B)** Flow cytometry analysis of human CD45⁺ cells in spleen in a GVHD humanized mouse model confirming the reduced infiltration of xenogenic donor leukocyte in this immune organ.

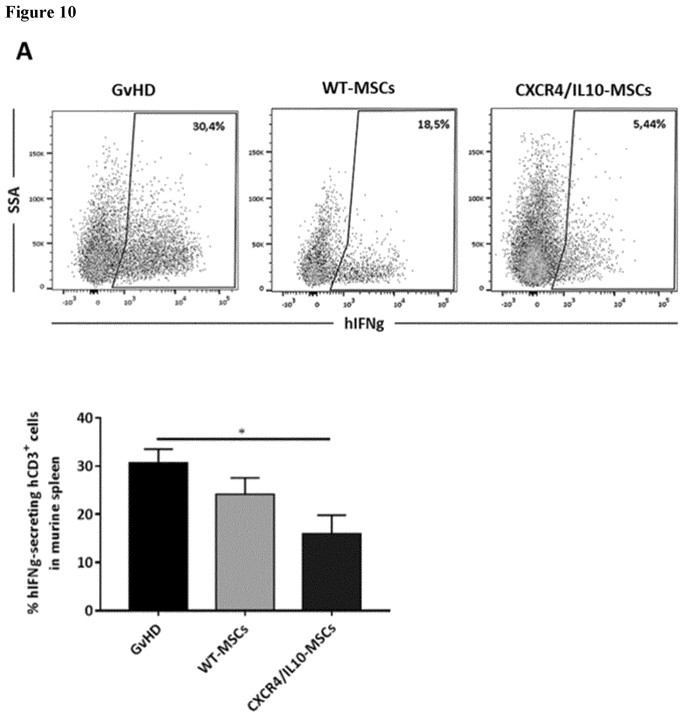

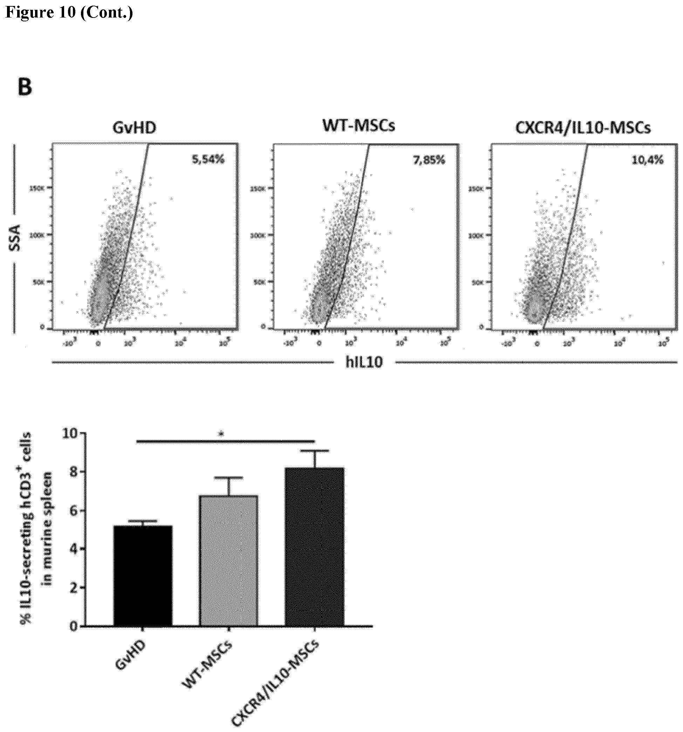

**Figure 10****. Enhanced anti-GvHD of MSCs transduced with the PGK-CXCR4-IL10 LV compared to WT-MSCs: Analysis of the infiltration of donor lymphocytes expressing IFN-g or IL10: A)** Reduced content of INFg-secreting human T cells responsible for GVHD disease in the spleen of NSG mice that had been infused with CXCR4-IL10-MSCs. **B)** Increased content of IL10-secreting human T cells in the spleen of NSG mice with GVHD treated with CXCR4-IL10-MSCs.

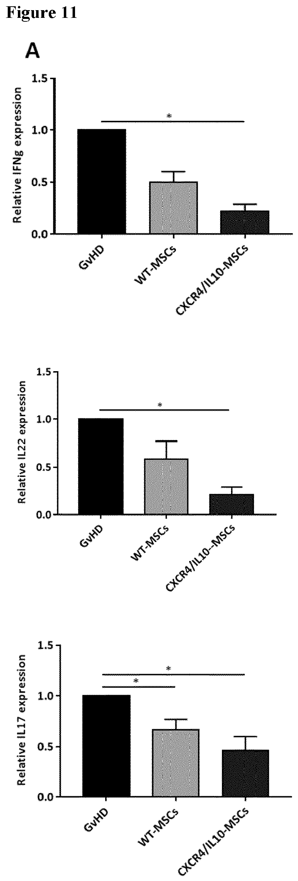

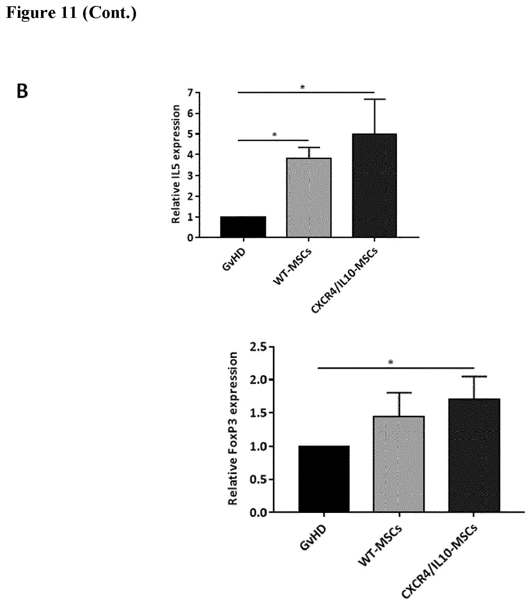

**Figure 11****. Enhanced anti-GvHD of MSCs transduced with the PGK-CXCR4-IL10 LV compared to WT-MSCs: Quantification of human factors in recipient mice by qPCR. A)** Analysis of pro-inflammatory factors (IFNg, IL-17 and IL-22) in the spleen of NSG treated with WT-MSC or CXCR4/IL10-MSCs. **B)** Analysis of anti-inflammatory factors (IL-5 or FoxP3) in the spleen of NSG treated with WT-MSC or CXCR4/IL10-MSCs.

### Detailed description of the invention

The present invention is illustrated by means of the examples set below without the intention of limiting its scope of protection.

### Example 1. Material and methods.

### Example 1.1. Generation and expansion of Adipose-derived MSCs (Ad-MSCs).

Adipose tissue samples were obtained by surgical resection from healthy donors after informed consent. Adipose tissue was disaggregated and digested with collagenase A (Serva, Germany) at a final concentration of 2 mg/ml for 4 hours at 37°C. Digested samples were filtered through 100 µm nylon filters (BD Bioscience, USA) and centrifuged for 10 minutes. The cell pellet was re-suspended in α-MEM (Gibco, USA) supplemented with 5% platelet lysate (Cook medical, USA), 1% penicillin/streptomycin (Gibco) and 1ng/ml human basic fibroblast growth factor (bFGF, Peprotech, USA). Cells were seeded at a concentration of 10,000 cells/cm2 in culture flasks (Corning, USA) and cultured at 37°C. For the expansion of Ad-MSCs, cell medium was changed every 2-4 days and adherent cells were serially passaged using 0.25% trypsin/EDTA (Sigma-Aldrich, USA) upon reaching near confluence (70%-90%). For in vitro and in vivo studies, Ad-MSCs were used at passages from 4 to 8.

### Example 1.2. Characterization of WT-MSCs and CXCR4/IL10-MSCs.

WT-MSCs and MSC that had been transduced with the CXCR4-IL10 lentiviral vector (CXCR4/IL10-MSCs) were immunophenotypically characterized by flow cytometry (Fortessa, BD Bioscience, USA) as described by the Mesenchymal cell kit (Immunostep, Spain). The monoclonal anti-human antibodies included in these studies were the following: CD29, CD44, CD73, CD90, CD105, CD166, CD45, CD19, HLA-DR, CD14 and CD34. Data were analysed with FlowJo version X (FlowJo LLC, USA).

The osteogenic and adipogenic differentiation ability of Ad-MSCs was determined using the NH-OsteoDiff and NH-AdipoDiff Media (Miltenyi Biotec, Germany), respectively, according to manufacturer's protocols. Alcaline phosphatase deposits were seen after the staining with Fast BCIP/NCP (Sigma-Aldrich) while lipid droplets were seen with optic microscopy (Nikon, Germany).

### Example 1.3. Construction of the CXCR4/IL10 lentiviral vector.

The fragment containing the lentiviral backbone and the PGK promoter (7362 bp) was obtained by simultaneous digestion of pCCL.PGK.FANCA.Wpre* plasmid (9087 bp) with Agel and SacII restriction enzymes (New England Biolabs, USA), whose restriction sites were blanking FANCA transgene at 5' and 3'-end, respectively. Digested lentiviral backbone without transgene was purified from agarose gel with NucleoSpin Gel and PCR Clean-up kit (Macherey-Nagel, Germany). Fragments containing codon-optimized sequences of human CXCR4 and IL10 were obtained by polymerase chain reaction (PCR) taking pUC57 plasmids used for mRNA synthesis as a template. PCR of each plasmid was performed using two specific primers which included Agel and SacII restriction sites at 5'-end and the first or last 20 bp of the CXCR4 or IL10 transgenes. Amplification was carried out following Herculase II Fusion Enzyme's protocol (Agilent, USA) depending on target size, without using dimethyl sulfoxide (DMSO) and stablishing 58°C for annealing temperature. PCR products were simultaneous digested with Agel and SacII and also purified by column using NucleoSpin Gel and PCR Clean-up kit.

Digested lentiviral backbone and fragments of interest were ligated with the T4 DNA Ligase (New England Biolabs) maintaining target:vector ratio at 5:1. Ligated products were transformed into Stable3 bacteria to obtain pCCL.PKG-CXCR4-IL10.Wpre*plasmid.

### Example 1.4. Lentiviral Vector production.

All self-inactivating HIV-1-derived vectors used in this work were produced by a second-generation packaging system in HEK293T cells, obtaining VSV-G-pseudotyped viruses. A total amount of 12x106 cells were plated the day before in 150mm diameter plates. Transfections were performed on cells at 70-80% confluence in 150mm diameter plates following the CaCl₂ DNA precipitation methods previously described. Briefly, one hour before transfection culture medium was replaced by fresh DMEM-Glutamax containing 10% HyClone (GE Healthcare, USA) and 1% penicillin/streptomycin. Equimolecular mixtures of three plasmids containing the transgenes, the viral genome and the packaging constructs were prepared freshly. HEK293T cells of each plate were transfected with 22,5µg of the gene transfer plasmid, 12 µg of the pMD2.VSVg envelope plasmid (PlasmidFactory, Germany) carrying the heterologous VSVg envelope and 27,5 µg of the pCMVdR8.74 packaging plasmid (PlasmidFactory) carrying the gag-pol-rev viral genes. These plasmid mixtures were prepared in a final volume of 3,8ml of ultra-pure H₂0 and 450µl of 2.5M CaCl2 were carefully added. After a 5 min incubation at room temperature, 3,8ml of 2X Hank's Buffered Saline (HBS) buffer (100mM HEPES (Gibco), 281mM NaCl, 1.5mM Na2HPO4, pH=7.13) was added drop by drop, allowing the formation of Ca2+ precipitates. This solution was added to HEK293T cells that would integrate those precipitates. Five hours after, medium containing precipitates was replaced by fresh medium. Supernatants were collected at 48 hours post-transfection. They were harvested, filtered using a 0.22 µm pore-size filter (Millipore, Merck KGaA, Germany) and concentrated by ultra-centrifuging at 20,000 rpm and 4°C for 2 hours. Then, viral pellets were suspended in DMEM for at least 1 h at 4°C, spun down to discard cellular debris and stored at -80°C in aliquot of 100 µl.

### Example 1.5. Transduction of the Ad-MSCs.

Two different strategies were carried out to transduce human Ad-MSCs: transduction of adhered MSCs and transduction of MSCs in suspension. In this set of experiments, transduction enhancers (TEs) were added during the transduction process with the aim of increasing the transduction efficacy.

### Example 1.6. CXCR4 and IL-10 protein co-expression.

The expression of CXCR4 on the cell surface of Ad-MSCs was determined by flow cytometry after labelling with a PE-conjugated anti-human CXCR4 antibody for 30 min at 4°C (Biolegend, USA). IL10 levels secreted by Ad-MSCs were measured in the supernatant of cultured cells using the human IL10 Quantikine ELISA Kit (R&D System, USA).

Total protein extracts were isolated from Ad-MSCs using the RIPA buffer (ThermoFisher Scientific, USA) containing a protease inhibitor mixture (Merck Millipore, Germany). Twenty micrograms of each of the cell lysates were resolved in 4-12% polyacrylamide gels (Bio-Rad, USA) and transferred to PVDF membranes (Bio-Rad). Membranes were blocked with 5% v/v nonfat dry milk in 0.1% Tween-20 PBS. Samples were immunoblotted by incubation with rabbit monoclonal anti-human CXCR4 antibody (Abcam, UK) diluted in blocking solution. Mouse anti-human Vinculin (Abcam) was used as a loading control. Blots were visualized with Clarity Western ECL substrate (Bio-Rad) using a ChemiDoc MP System and ImageLab sofware (Bio-Rad).

### Example 1.7. Cell migration assay.

Migration assays were carried out in transwells with an 8 µm pore polycarbonate membrane insert (Costar, Cambridge, MA). 5x103 Ad-MSCs were placed in the upper insert chamber of the transwell assembly. The lower chamber contained murine or human SDF-1 (Peprotech, USA) at a final concentration of 100 ng/ml. Twenty-four hours after incubation, the upper part of the membrane was scrapped gently by a cotton swab to remove non-migrating cells and washed with PBS. The membrane was fixed with 3.7-4% formalin overnight at 4°C and stained with haematoxylin for 4 hours at RT. The number of migrating cells was determined by the scoring of four random fields per well under the Nikon Eclipse E400 microscope (10X) (Nikon, UK) and pictures were obtained with a Leica DFC420 camera (Leica, UK).

### Example 1.8. In vitro immunosuppression assay.

Peripheral blood mononuclear cells (MNCs) were obtained by Ficoll-Paque PLUS (GE Healthcare Bioscience, Sweden) density gradient from heparinized peripheral blood samples obtained from healthy donors after informed consent. Before co-culture, MNCs were marked with the intracellular fluorescent dye CFSE (carboxyfluorescein diacetate succinimidyl ester, Molecular Probe, USA), following a previously described protocols. WT-MSCs and CXCR4/IL10-MSCs were plated in 24-well plates at a concentration of 5x104 cells/well. Twenty-four hours later, 5x105 MNCs were added to each well in presence of 10 µg/mL of phytohemagglutinin (PHA) (Sigma-Aldrich) to induce the T cell proliferation. After 3 days of incubation, cells harvested from culture wells were analysed by flow cytometry for cell proliferation. Data were analysed with ModFit LT^{™} (Verity Software House, USA).

### Example 1.9. Quantification of secreted cytokines and factors.

WT-MSCs and CXCR4/IL10-MSCs were seeded in 6-well plates at a concentration of 1x105 cells/well. At 4h post-transfection, supernatants were collected and secreted PGE2 and TGFβ1 were quantified by ELISA (R&D System, USA). Secreted IL-6, IFNγ and TNFα were quantified by flow cytometry using LEGENDplex^{™} Human Th Cytokine Panel (Biolegend, USA) following manufacturer's protocol.

### Example 1.10. Gene expression analysis.

RNA from WT-MSCs and CXCR4/IL10-MSCs was isolated using RNAeasy^{®} Plus Mini Kit and reverse transcribed with RETROscript (ThermoFisher Scientific, Waltham, USA). cDNA was subjected to quantitative Real-Time PCR (qPCR) using FastStart Universal SYBR Green Master master mix (Roche, Indianapolis, USA) and specific primers for human interleukins and different factors. qPCRs were run on a 7,500 fast real-time PCR system (ThermoFisher Scientific). Results were normalized to human GAPDH expression and expression of control samples according to the 2^{-ΔΔCt} method.

### Example 1.11. LPS-induced inflamed pad model.

FVB/NJ mice were housed in the animal facility (Registration No. ES280790000183) at CIEMAT (Madrid, Spain). Mice were routinely screened for pathogens in accordance with FELASA procedures and received water and food ad libitum. All experimental procedures were carried out according to Spanish and European regulations (Spanish RD 53/2013 and Law 6/2013, European Directive 2010/63/UE). Procedures were approved by the CIEMAT Animal Experimentation Ethical Committee according to approved biosafety and bioethics guidelines. FVB/NJ mice were sedated and administered a single injection of 40 µg of E. coli LPS in 30 µl of PBS into the right pad. Similarly, 30 µL of PBS were injected into the left pad, as a control. Four hours after Ad-MSCs transfection, 5x105 WT-MSCs or CXCR4/IL10-MSCs were intravenously infused through the tail vein. Pad inflammation was determined by measuring the thickness with a digital calliper at 24, 48 and 72 h following LPS administration. At the end of the experiments, mice were sacrificed by CO₂ inhalation. Peripheral blood cells were collected to analyse the mouse haematological parameters using the hematology analyzer Abacus (Diatron, USA).

### Example 1.12. Humanized mouse model of graft-versus-host disease (GvHD) in NSG mice.

To establish the model, NSG mice were irradiated with 2Gy and the following day they were transplanted with 5x10⁶ human MNCs. Three days later, one million of WT-MSCs or CXCR4/IL10-MSCs were infused intravenously. Animals were weighed daily and monitored for possible symptoms of GVHD such as weight loss, hunched back, ruffling of hair and diarrhea. The severity of GVHD was graded from 0 (absence of GVHD) to 8 (severe GVHD). Animals were sacrificed humanely when they exhibited the euthanasia GVHD criteria (>20 % weight loss or a score ≥ 6.5).

### Example 1.13. Statistical analysis.

Statistical analyses were performed using Graph Pad Prism 7.0 software (Graph Pad Software, USA). Data of in vitro tests are expressed as mean ± standard deviation (SD) and as mean ± the standard error of the mean (SEM) in in vivo tests. Normal distribution was analyzed by the Shapiro-Wilks test. To compare more than two groups, parametric test (one-way ANOVA) for normal distribution and non-parametric test (Kruskal-Wallis test) for non-normal distribution were used. Appropriate post hoc analysis to compare means was performed. P values < 0.05 were considered statistically significant.

### Example 2. Results.

For the sake of clarity, kindly note that the results provided in **Examples 2.1** and **2.2,** with respect to MSCs transfected with bicistronic CXCR4-IL10 mRNA, are just included as comparative examples to show how these results were improved when the MSCs were transduced with an integrative expression vector co-expressing CXCR4 and IL10 **(Examples 2.3** to **2.7).**

### Example 2.1. MSCs transfected with the bicistronic CXCR4-IL10 mRNA exhibit significant local anti-inflammatory effects.

We tested the *in vivo* efficacy of MSCs transfected with the bicistronic CXCR4-IL10 mRNA in a mouse model of local inflammation model induced by LPS. Both WT and CXCR4-IL10-RNA-MSCs were able to exhibit significant anti-inflammatory effects, although MSCs transfected with the bicistronic CXCR4-IL10 mRNA were significantly more efficient compared to WT-MSCs. See **Figure 1** wherein it is shown the analysis of the *in vivo* efficacy of MSCs transfected with the bicistronic CXCR4-IL10 mRNA in a mouse model of local inflammation. Enhanced anti-inflammatory effect of MSCs transfected with the CXCR4-IL10 mRNA is observed as compared to WT-MSCs.

### Example 2.2. Absence of in vivo efficacy of MSCs transfected with the bicistronic CXCR4-IL10 mRNA in a graft versus host disease mouse model.

We also tested the *in vivo* efficacy of MSCs transfected with the bicistronic CXCR4-IL10 mRNA in a graft versus host disease mouse model. A mouse model of haploidentical hematopoietic transplantation was conducted by transplanting BM cells from C57Bl/6 donor mice into B6D2F1 recipient mice, previously irradiated with a lethal dose of 11 Gy. All recipients were injected intravenously with 10x10⁶ BM donor cells. To induce graft versus host disease (GVHD), recipients also received a total number of 2x10⁸ donor splenocytes. One day after GVHD induction, mice were administered saline (GVHD group), WT-MSCs or mRNA-transfected MSCs (1×10⁶) via the tail vein. Transplanted recipients were observed daily for symptoms of GVHD such as weight loss, hunched back, ruffling of hair and diarrhea. The severity of GVHD was graded from 0 (absence of GVHD) to 8 (severe GVHD). Animals were sacrificed humanely when they exhibited the euthanasia GVHD criteria (>20 % weight loss or a score ≥ 6.5). **Figure 2** shows the analysis of the *in vivo* efficacy of MSCs transfected with the bicistronic CXCR4-IL10 mRNA in a mouse model of GVHD. A) Survival curve B) Weight and C) Clinical score. As shown in **Figure 2A****,** we did not observe any difference between the WT-MSCs and CXCR4-IL10 mRNA MSCs to inhibit GVHD

### Example 2.3. Generation of MSCs transduced with a bicistronic CRCR4-IL10 lentiviral vector for improving the efficacy of WT MSCs to inhibit graft versus host disease

In these studies, we have generated a lentiviral vector in which the optimized sequences of the CXCR4 and IL10 genes have been cloned in a bicistronic lentiviral vector under the human physiological promoter PGK **(****Figure 3A****).**

After testing different methods of Ad-MSCs transduction as well as different amounts of the vector, a population of modified Ad-MSCs (CXCR4/IL10-MSCs) was obtained. This population of CXCR4/IL10-MSCs overexpressed CXCR4, around 80% MSCs was positive to CXCR4. Higher concentrations of IL10 were secreted by CXCR4/IL10-MSCs compared to unmodified MSCs (WT-MSCs). The vector copy number was analyzed in these CXCR4/IL10-MSCs by qPCR **(****Figure 3B****).**

### Example 2.4. In vitro characterization of CXCR4/IL10-MSCs compared to WT-MSC.

MSCs modified with the bicistronic PGK-CXCR4-IL10 lentiviral vector were characterized following the criteria established by the ISCT (International Society of Cellular Therapy) for mesenchymal cells.

The *in vitro* characterization showed that the modification of the MSCs with the bicistronic lentiviral vector did not affect their immunophenotype **(****Figure 4A****)** nor their ability to differentiate to bone **(****Figure 4B****)** and adipose **(****Figure 4C****)** tissue compared to unmodified mesenchymal cells (WT-MSCs).

### Example 2.5. In vitro functionality of CXCR4/IL10-MSCs compared to WT-MSC.

To study the *in vitro* functionality of mesenchymal cells modified with the bicistronic lentiviral vector (CXCR4/IL10-MSCs), a transwell migration assay was first performed in response to SDF-1, ligand of CXCR4 **(****Figure 5A****).** The results of this assay showed an enhanced migration ability of CXCR4/IL10-MSCs as compared to WT-MSCs **(****Figure 5B****).**

The second *in vitro* functional characterization study consisted of an immunosuppression assay in which the ability of the CXCR4/IL10-MSCs to inhibit the proliferation of activated mononuclear cells (MNCs) was evaluated compared to WT-MSCs **(****Figure 6A****).**

As already described, WT-MSCs showed a high capacity to inhibit the proliferation of activated MNCs. However, this inhibition was significantly higher when MSCs were transduced with the PGK-CXCR4-IL10 lentiviral vector **(****Figure 6B****).** These studies demonstrate that the transduction of MSCs with the bicistronic lentiviral vector significantly improve the immunomodulatory capacity of these cells compared to WT-MSCs.

### Example 2.6. Enhanced in vivo efficacy of CXCR4/IL10-MSCs to inhibit local inflammation compared to WT-MSC.

To test the *in vivo* efficacy of MSCs transduced with the PGK-CXCR4-IL10 lentiviral vector, cells were tested in a mouse model of local inflammation model induced by LPS.

The LPS was injected on the right pad of each mouse. One day after LPS injection, the different types of Ad-MSCs (WT-MSCs and CXCR4/IL10-MSCs) were infused intravenously (n = 7-14 mice / group). Inflammation was measured macroscopically with a digital caliper, using the left pad as a control in each mouse **(****Figure 7A****).**

The results showed that 24 hours after the infusion of the Ad-MSCs (48 hours after the LPS injection), all the mice that had received Ad-MSCs controlled the inflammation, while the inflammation continued to grow in the group of mice that had only received the LPS injection.

However, the control of the inflammation was statistically higher in the group of mice that had received CXCR4/IL10-MSCs **(****Figure 7B****).**

### Example 2.7. Improved efficacy of MSCs transduced with the bicistronic lentiviral vector to inhibit graft-versus-host disease (GvHD) compared to WT MSCs.

The therapeutic efficacy of MSCs transduced with the bicistronic lentiviral vector was also tested in a graft-versus-host disease (GvHD) mouse model based on the infusion of peripheral blood human mononuclear cells (MNC) in immunodeficient NSG mice **(****Figure 8A****).** To establish the model, mice were irradiated with 2Gy and the following day they were transplanted with 5x10⁶ human MNCs. Three days later, one million of WT-MSCs or CXCR4/IL10-MSCs were infused intravenously. Animals were weighed daily and monitored for possible key signs of GVHD **(****Figure 8B****).**

As **Figure 8B** shows, GVHD score was significant better in the group of NSG mice that received CXCR4/IL10-MSCs, comparing not only with GVHD groups but also WT-MSCs group.

Two weeks after the infusion of MNCs, mice that only received human MNCs (GvHD group) began to show signs of the disease (weight loss, hunched back). Therefore, at this time recipient mice from all the three groups were sacrificed to analyze the percentage of human CD45⁺ cells in the peripheral blood (PB) and in the spleen (SP). It was found that the percentage of infiltrating human CD45⁺ cells was significantly reduced in mice that received WT-MSCs. Nevertheless, the reduction observed both in PB and spleen was significantly higher in mice that were infused with CXCR4/IL10-MSCs **(****Figure 9A-B****)**.

Human CD45⁺CD3⁺ cells responsible for GVHD disease were analyzed by flow cytometry in the GVHD humanized mouse model. Remarkably, NSG mice treated with CXCR4/IL10-MSCs, but not with WT-MSCs, showed a statistically reduced percentage of pro-inflammatory T cells (CD3⁺IFNg⁺) compared to the GvHD control group **(****Figure 10A****).** In addition, a statistically significant increase in the percentage of anti-inflammatory T cells (CD3⁺IL10⁺) was also observed **(****Figure 10B****)** in the group that received CXCR4/IL10-MSCs, but not with WT-MSCs, compared to the GvHD control group.

These data observed by flow cytometry were confirmed by qPCR. Pro-inflammatory factors such as IFNg, IL-17 and IL-22 were significantly reduced in the case of mice that received CXCR4/IL10-MSCs, but not WT-MSCs, compared to the control GvHD group. Quantification of the levels of expression of anti-inflammatory factors such as IL-5 or FoxP3 showed that these factors were statistically increased in the case of mice that received CXCR4/IL10-MSCs, but not WT-MSCs, with respect to the control GvHD group **(****Figure 11****).**

## Claims

1. An expression cassette comprising a polynucleotide sequence comprising:
a. A promoter;
b. A sequence encoding the chemokine receptor type 4 CXCR4; and
c. A sequence encoding interleukin IL-10.

2. The expression cassette, according to claim 1, further comprising a regulatory element for increasing transgene expression.

3. The expression cassette, according to claim 2, wherein the regulatory element is the woodchuck hepatitis virus regulatory element (WPRE) RNA export signal sequence or a functional variant or fragment thereof.

4. The expression cassette, according to claim 1, further comprising, between the sequence encoding the chemokine receptor type 4 CXCR4 and the sequence encoding interleukin IL-10, a sequence which encodes an autocatalytic peptide.

5. The expression cassette, according to claim 4, wherein the autocatalytic peptide is E2A.

6. The expression cassette, according to claim 1, wherein the promoter is a human phosphoglycerate kinase (PGK) promoter sequence or a functional homolog or variant thereof.

7. The expression cassette, according to any of the claims 1 to 6, comprising in the order 5' to 3':
a. A human phosphoglycerate kinase (PGK) promoter sequence or a functional homolog or variant thereof;
b. A sequence encoding the chemokine receptor type 4 CXCR4;
c. A sequence encoding the autocatalytic peptide E2A;
d. A sequence encoding interleukin IL-10; and
e. The woodchuck hepatitis virus post-transcriptional regulatory element (WPRE).

8. A recombinant gene delivery integrative vector comprising the expression cassette of any of the claims 1 to 7.

9. The recombinant gene delivery integrative vector of claim 8 **characterized in that** it is a lentiviral vector.

10. A cell comprising the expression cassette of any of the claims 1 to 7 or the recombinant gene delivery integrative vector of any of claims 8 or 9.

11. The cell, according to claim 10, **characterized in that** it is a mesenchymal stem cell derived from bone marrow, placenta, umbilical cord, amniotic membrane, menstrual blood, peripheral blood, salivary gland, skin and foreskin, synovial fluid, amniotic fluid, endometrium, adipose tissue, cord blood and / or dental tissue.

12. A pharmaceutical composition comprising the recombinant gene delivery integrative vector of any of claims 8 or 9 or the cell of any of the claims 10 or 11 and, optionally, a pharmaceutically acceptable excipient or carrier.

13. Gene delivery integrative vector of any of claims 8 or 9 or the cell of any of the claims 10 or 11 for use as medicament.

14. Gene delivery integrative vector of any of claims 8 or 9 or the cell of any of the claims 10 or 11 for use, according to claim 13, in the treatment of inflammatory diseases.

15. Gene delivery integrative vector of any of claims 8 or 9 or the cell of any of the claims 10 or 11 for use, according to any of the claims 13 or 14, in the treatment of Graft-versus-host disease (GvHD), sepsis or rheumatoid arthritis.
